Europäisches Patentamt

**European Patent Office**

Office européen des brevets

(11) Veröffentlichungsnummer: **0 257 118**
**B1**

(12) **EUROPÄISCHE PATENTSCHRIFT**

(45) Veröffentlichungstag der Patentschrift:
13.06.90

(51) Int. Cl.⁵: **A61B 17/58**

(21) Anmeldenummer: **86111628.3**

(22) Anmeldetag: **22.08.86**

(54) Osteosynthesehilfsmittel zur Versorgung subtrochanterer Frakturen.

(30) Priorität: **30.07.86 DE 8620399 U**

(43) Veröffentlichungstag der Anmeldung:
**02.03.88 Patentblatt 88/9**

(45) Bekanntmachung des Hinweises auf die Patenterteilung:
**13.06.90 Patentblatt 90/24**

(84) Benannte Vertragsstaaten:
**AT BE CH DE FR GB IT LI LU NL**

(56) Entgegenhaltungen:
**DE-A- 2 854 334**
**DE-A- 2 854 334**
**US-A- 3 996 931**
**US-A- 3 996 931**

(73) Patentinhaber: **Howmedica GmbH,**
**Professor-Küntscher-Str. 1-5, D-2314 Schönkirchen ü.**
**Kiel(DE)**

(72) Erfinder: **Grosse, Arsêne, Dr., 19 rue Boussingault,**
**F-67000 Strassburg(FR)**
Erfinder: **Harder, Hans Erich, Mecklenburger Strasse 35,**
**D-2316 Probstelerhagen(DE)**

(74) Vertreter: **Dipl.-Ing. H. Hauck Dipl.-Phys. W. Schmitz**
**Dipl.-Ing. E. Graalfs Dipl.-Ing. W. Wehnert Dr.-Ing. W.**
**Döring, Neuer Wall 41, D-2000 Hamburg 36(DE)**

## Beschreibung

Die Erfindung bezieht sich auf ein Osteosynthesehilfsmittel zur Versorgung subtrochanterer Frakturen, mit einem in den Markraum einführbaren Femurnagel und einer von dem Femurnagel geführten Schenkelhalsschraube, die von lateral in den Femurhals einführbar ist. Ein derartiges Osteosynthesehilfsmittel ist bekannt. Der als im Querschnitt V-förmig flach ausgeführte Subtrochanterstift weist eine Bohrung auf, durch die von distal ein Marknagel nach Küntscher hindurchgeführt wird. Bei der Operation bereitet es gewisse Schwierigkeiten, den Marknagel durch das Loch im Subtrochanterstift hindurchzufädeln. Hierzu ist erforderlich, daß beim Einschlagen des Subtrochanterstifts das Loch richtig ausgerichtet ist und der Subtrochanterstift nicht zu wenig oder zu weit eingetrieben ist. Nachteilig ist schließlich, daß der Subtrochanterstift zwar gegen Drehung gesichert ist, jedoch zur Seite verschwenken kann und daher nicht in besonderem Maße zur Stabilisierung der Fraktursegmente beiträgt.

Es ist ferner aus DE-A 2 854 334 bekannt, einen mit selbstschneidendem Gewinde versehenen Trochanterstift von lateral in den Femurhals und den Kopf einzuschrauben, wobei der Stift durch eine in eine Bohrung eingeführte Hülse hindurchgesteckt ist. Die Hülse wird mittels einer Lasche und die Lasche mittels Knochenschrauben am Femur fixiert. Ein derartiges Osteosynthesehilfsmittel, das auch als Pohl'sche oder Seidellasche bekannt ist, hat sich im Prinzip bewährt. Die Befestigung der Lasche kann zu einer Schwächung des Femurs führen. Ferner ist durch die einmal gewählte Lage der Bohrung, in die die Hülse der Lasche eingeführt wird, auch die Lage des Subtrochanterstiftes vorgegeben.

Der Erfindung liegt die Aufgabe zugrunde, ein Osteosynthesehilfsmittel zur Versorgung subtrochanterer Frakturen zu schaffen, das einen geringen operativen Aufwand erfordert und mit dem während der Operation die Lage der Schenkelhalsschraube auf einfache Weise bestimmt werden kann.

Diese Aufgabe wird neuerungsgemäß dadurch gelöst, daß der Femurnagel ein proximal in den Femur einführbarer, an sich bekannter Verriegelungsnagel ist, der mindestens eine Querdurchbohrung aufweist für die Aufnahme einer Knochenschraube, die von lateral durch die Querdurchbohrung einführbare, im Endabschnitt ein mit dem Schenkelhals bzw. dem Femurkopf zusammenwirkendes Gewinde aufweisende Schenkelhalsschraube in der Querdurchbohrung frei axial verschiebbar ist und am Verriegelungsnagel eine Drehsicherung angeordnet ist, die wahlweise eine Drehung der Schenkelhalsschraube verhindert, eine axiale Bewegung jedoch weiterhin zuläßt. Verriegelungsnägel sind an sich bekannt und werden zur Versorgung von Brüchen, zum Beispiel im Oberschenkel- oder Unterschenkelbereich verwendet. Ein Verriegelungsnagel ist verhältnismäßig biegsteif und füllt nahezu den Markraum eines Knochens aus. Er besitzt mehrere Querbohrungen. Mit Hilfe von Knochenschrauben beidseitig der Fraktur wird der Verriegelungsnagel im Knochen fest verankert ("verriegelt"). Der Knochen ist nach der Versorgung mit einem Verriegelungsnagel innerhalb rascher Zeit wieder belastbar. Beim erfindungsgemäßen Osteosynthesehilfsmittel dient der Verriegelungsnagel nicht selbst zur Frakturversorgung, sondern zur Führung und Halterung der Schenkelhalsschraube. Der Verriegelungsnagel weist daher lediglich im distalen Bereich Querbohrungen auf, um den Nagel axial und in Drehrichtung festzulegen. Im proximalen Bereich weist er eine schräge Durchbohrung auf, durch die die Schenkelhalsschraube hindurchführbar ist.

Verriegelungsnägel werden normalerweise mit Hilfe eines sogenannten Einschlaginstruments eingesetzt. Mit Hilfe dieses Instruments ist es möglich, die Position sowohl in Achs- als auch in Drehrichtung zu bestimmen. Mit Hilfe eines Zielgeräts kann genau die Lage der schrägen Durchbohrung bestimmt werden. Der Operatur weiß daher, wo er lateral den Femur anbohren muß, um die Schenkelhalsschraube einzuführen und in den Femurhals und -kopf einzuschrauben.

Die Schenkelhalsschraube sitzt vorzugsweise passend in der schrägen Durchbohrung. Die Schenkelhalsschraube ist daher sowohl gegen ein Verschwenken in der von ihr aufgespannten Ebene als auch um eine durch den Verriegelungsnagel gehende Achse wirksam gesichert. Es findet mithin eine optimale Stabilisierung der Fraktursegmente statt.

Ein wesentliches Merkmal der Erfindung sieht vor, daß eine Drehsicherung der Schenkelhalsschraube in der Schrägdurchbohrung vorgesehen ist, die eine axiale Bewegung der Schenkelhalsschraube zuläßt. Während der Knochenheilung kommt es zumeist zu einer sogenannten Sinterung, wodurch sich der Knochen im Bereich der Fraktur verkürzt. Würde die Schenkelhalsschraube dieser Verkürzung nicht nachgeben, besteht einerseits die Gefahr, daß die Schenkelhalsschraube den Femurkopf durchstößt, andererseits die Fekturversorung unstabil wird. Diese Gefahr ist zum Beispiel bei dem eingangs beschriebenen Y-Nagel nach Küntscher der Fall, der durch den Marknagel an einer Axialbewegung hindert ist.

Es sind verschiedene konstruktive Möglichkeiten denkbar, die Schenkelhalsschraube in Drehrichtung zu sichern und eine axiale Bewegung zuzulassen. Eine besteht erfindungsgemäß darin, daß im Bereich der Schrägdurchbohrung am Umfang der Schenkelhalsschraube mehrere achsparallele, in Umfangsrichtung beabstandete Nuten geformt sind, in die ein in das proximale Ende des Verriegelungsnagels einsetzbarer Verriegelungsstift eingreift. Die Nuten lassen eine Axialbewegung der Schenkelhalsschraube zu. Da der Verriegelungsstift in eine der Nuten eingreift, verhindert sie, daß die Schenkelhalsschraube sich ungewollt verdrehen kann.

Der Verriegelungsnagel kann beim erfindungsgemäßen Osteosynthesehilfsmittel kürzer als üblich sein. Eine Länge zwischen 150 und 250 mm, vorzugsweise 200 mm, reicht aus, um für eine sichere Halterung der Schenkelhalsschraube zu sorgen. Der proximale Bereich des Verriegelungsnagels ist mit einer verhältnismäßig großen Wanddicke ausgeführt, die größer ist als bei herkömmlichen Verriegelungsnägeln, was zu günstigen Flächenpressungswerten zwischen Schenkelhalsschraube und Nagel führt.

Die Schenkelhalsschraube weist vorzugsweise im Endbereich ein selbstschneidendes Gewinde auf, wobei an der Spitze zwei diametral gegenüberliegende scharfkantige hinterschnittene Schneiden geformt sind.

Die Erfindung wird nachfolgend anhand von Zeichnungen näher erläutert.

Fig. 1 zeigt schematisch teilweise im Schnitt das Osteosynthesehilfsmittel nach der Erfindung.

Fig. 2 zeigt einen Schnitt durch den distalen Teil des Verriegelungsnagels des Osteosynthesehilfsmittels nach Fig. 1 entlang der Linie 2–2.

Fig. 3 zeigt den distalen Bereich des Verriegelungsnagels nach Fig. 1 um 90° gedreht in Richtung Pfeil 3.

Fig. 4 zeigt einen Schnitt durch das vordere Ende der Schenkelhalsschraube des Osteosynthesehilfsmittels nach Fig. 1 entlang der Linie 4–4.

Fig. 5 zeigt einen Schnitt durch den lateralen Bereich der Schenkelhalsschraube nach Fig. 1 entlang der Linie 5–5.

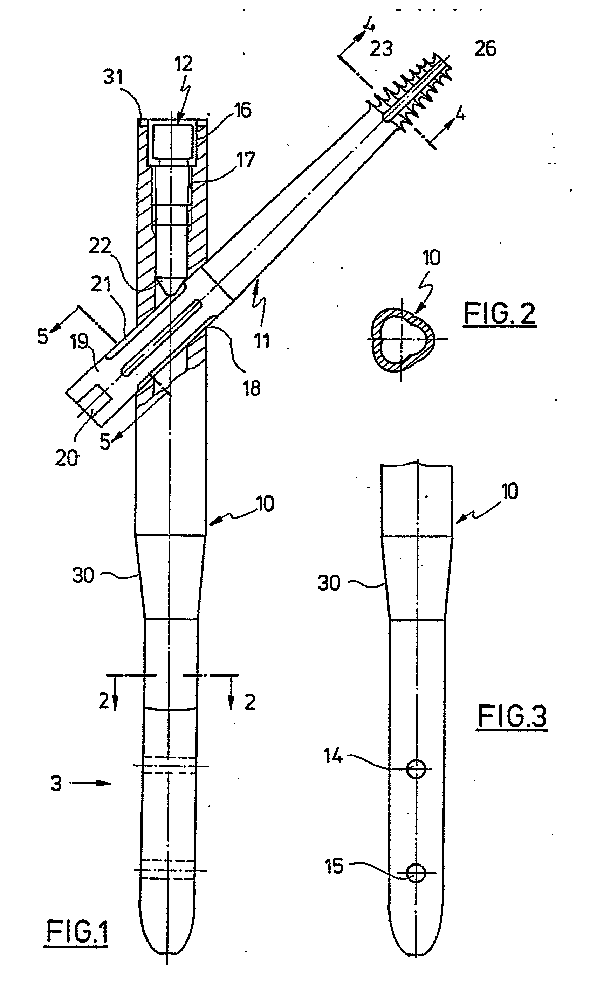

Das in Fig. 1 dargestellte Osteosynthesehilfsmittel weist einen Verriegelungsnagel 10, eine Schenkelhalsschraube 11 sowie einen Verriegelungsstift 12 auf. Aus Fig. 2 ist zu erkennen, daß der untere distale Abschnitt des Verriegelungsnagels 10 im Querschnitt einen dreiblättrigen geschlossenen Kleeblatt entspricht. Der obere proximale Abschnitt ist hingegen im Querschnitt kreisförmig. Der Durchmesser des unteren Bereichs ist kleiner als der des oberen Bereichs, wobei der distale Bereich zum Beispiel 15 mm und der proximale 19 mm beträgt. Ein Übergangsbereich 30 ist konisch ausgeführt. Wie aus Fig. 3 zu erkennen, besitzt der untere Bereich des Verriegelungsnagels 10 zwei Querdurchbohrungen 14, 15.

Wie aus Fig. 1 zu erkennen, hat der Verriegelungsnagel im proximalen Bereich eine verhältnismäßig große Wanddicke, die größer ist als der halbe Durchmesser der Innenbohrung. Das proximale Ende ist zylindrisch aufgeweitet, wie bei 16 dargestellt, der Rand ist bei 31 diametral geschlitzt. Zylindrische Aufweitung und Schlitze dienen zur Aufnahme und Positionierung eines Zielgeräts oder eines Einschlagwerkzeugs. Unterhalb der zylindrischen Aufweitung 16 ist ein Innengewinde 17 geformt. In das Innengewinde wird der Gewindeabschnitt des Verriegelungsstiftes 12 eingeschraubt.

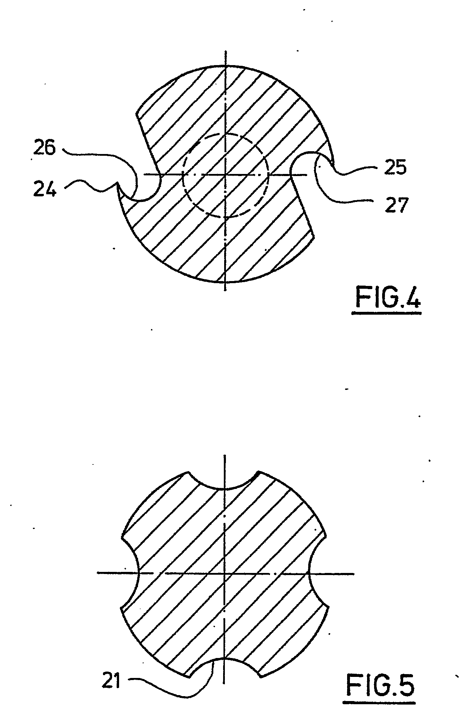

Am Ende des oberen Viertels des Verriegelungsnagels 11 ist eine Schrägbohrung 18 geformt. Durch die Schrägbohrung 18 ist die Schenkelhalsschraube 11 hindurchgeführt. Ihr zylindrischer Abschnitt 19 sitzt passend in der Bohrung 18. Im lateralen Ende weist die Schenkelhalsschraube 11 einen versenkten Schlitz 20 auf für den Ansatz eines Drehwerkzeugs. Im zylindrischen Abschnitt 19 sind mehrere achsparallele im Umfangsabstand gleichmäßig beabstandete Nuten 21 geformt. Das spitze abgerundete Ende 22 des Verriegelungstiftes 12 kann in eine der Nuten 21 eingreifen und dadurch die Schenkelhalsschraube 11 an einer Drehung hindern. Axial kann sich die Schenkelhalsschraube 11 hingegen bewegen. In Fig. 5 sind die Nuten 21 im Querschnitt gezeigt. Sie sind im Querschnitt kreisbogenförmig. Auf dem Umfang des zylindrischen Abschnitts 19 sind vier Nuten 21 geformt.

Die Schenkelhalsschraube 11 besitzt ein selbstschneidendes Gewinde 23. Fig. 4 zeigt einen Schnitt durch das Gewinde. Man erkennt zwei schräg eingeformte diametral gegenüberliegende Nuten 26, 27, die sich achsparallel erstrecken und zwei diametral gegenüberliegende scharfkantige Schneiden 24, 25 bilden.

**Patentansprüche**

1. Osteosynthesehilfsmittel zur Versorgung subtrochanterer Frakturen, mit einem in den Markraum einführbaren Femurnagel (10) und einer in einer Querdurchbohrung (18) des Femurnagels gehaltenen und geführten Schenkelhalsschraube (11), wobei der Femurnagel (10) ein proximal in den Femur einführbarer Verriegelungsnagel (10) ist, der mindestens eine Querdurchbohrung (14, 15) aufweist für die Aufnahme einer Knochenschraube, dadurch gekennzeichnet, daß die von lateral durch die Querdurchbohrung (18) einführbare, im Endabschnitt ein mit dem Schenkelhals bzw. dem Femurkopf zusammenwirkendes Gewinde aufweisende Schenkelhalsschraube (11) in der Querdurchbohrung (18) frei axial verschiebbar ist und am Verriegelungsnagel (10) eine Drehsicherung (12) angeordnet ist, die wahlweise eine Drehung der Schenkelhalsschraube (11) verhindert, eine axiale Bewegung jedoch weiterhin zuläßt.

2. Osteosynthesehilfsmittel nach Anspruch 1, dadurch gekennzeichnet, daß im Bereich der Schrägdurchbohrung (18) am Umfang der Schenkelhalsschraube (11) mehrere achsparallele, in Umfangsrichtung beabstandeten Nuten (21) geformt sind, in die ein in das proximale Ende des Verriegelungsnagels (10) festsetzbarer Verriegelungsstift (12) eingreift.

3. Osteosynthesehilfsmittel nach einem der Ansprüche 1 bis 2, dadurch gekennzeichnet, daß der Verriegelungsnagel (10) im proximalen Bereich hohl ist und im distalen Bereich massiv oder hohl ausgeführt ist.

4. Osteosynthesehilfsmittel nach Anspruch 3, dadurch gekennzeichnet, daß der distale Bereich einen etwas geringeren Durchmesser aufweist als der proximale Bereich.

5. Osteosynthesehilfsmittel nach einem der Ansprüche 1 bis 4, dadurch gekennzeichnet, daß der Verriegelungsnagel (10) im distalen Bereich im Querschnitt ein Kleeblattprofil aufweist.

6. Osteosynthesehilfsmittel nach einem der Ansprüche 1 bis 5, dadurch gekennzeichnet, daß die Länge des Verriegelungsnagels zwischen 150 und 250 mm, vorzugsweise etwa 200 mm, beträgt.

7. Osteosynthesehilfsmittel nach einem der Ansprüche 3 bis 6, dadurch gekennzeichnet, daß die Wanddicke im hohlen Bereich des Verriegelungsnagels (10) mindestens den halben Durchmesser der Innenbohrung entspricht.

8. Osteosynthesehilfsmittel nach einem der Ansprüche 2 bis 7, dadurch gekennzeichnet, daß der Verriegelungsstift (12) versenkt im Verriegelungsnagel (10) angeordnet ist.

9. Osteosynthesehilfsmittel nach einem der Ansprüche 1 bis 8, dadurch gekennzeichnet, daß die Schenkelhalsschraube (11) im Endbereich ein selbstschneidendes Gewinde (23) aufweist.

10. Osteosynthesehilfsmittel nach Anspruch 9, dadurch gekennzeichnet, daß im Gewindebereich der Schenkelhalsschraube zwei diametral gegenüberliegende scharfkantige hinterschnittene Schneiden (24, 25) geformt sind.

## Revendications

1. Dispositif d'ostéosynthèse pour le traitement de fractures subtrochantériennes, comportant un clou fémoral (10) pouvant être inséré dans la cavité médullaire, et une vis (11) destinée à être fixée dans le col du fémur, qui est maintenue et guidée dans un perçage traversant transversal (18) du clou fémoral, le clou fémoral (10) étant un clou de verrouillage (10), qui peut être inséré, du côté proximal, dans le fémur et présente au moins un perçage traversant transversal (14, 15) servant à loger une vis pour os, caractérisé en ce que la vis (11) destinée à être fixée dans le col du fémur, qui peut être insérée latéralement dans le perçage traversant transversal (18) et possède, dans sa section terminale, un filetage coopérant avec le col ou la tête du fémur, peut être déplacée axialement librement dans le perçage traversant transversal (18) et que, dans le clou de verrouillage (10), est installé un dispositif de blocage en rotation (12), qui, au choix, empêche une rotation de la vis (11) destinée à être fixée dans le col du fémur, tout en autorisant cependant un déplacement axial.

2. Dispositif d'ostéosynthèse selon la revendication 1, caractérisé en ce que dans la région du perçage traversant oblique (18), sont ménagées, sur le pourtour de la vis (11) destinée à être fixée dans le col du fémur, plusieurs rainures (21) parallèles à l'axe, espacées suivant la direction circonférentielle et dans lesquelles s'engage une tige de verrouillage (12) pouvant être fixée dans l'extrémité proximale du clou de verrouillage (10).

3. Dispositif d'ostéosynthèse suivant l'une des revendications 1 et 2, caractérisé par le fait que le clou de verrouillage (10) est creux dans sa partie proximale et est massif ou creux dans sa partie distale.

4. Dispositif d'ostéosynthèse selon la revendication 3, caractérisé en ce que la partie distale possède un diamètre légèrement inférieur à celui de la partie proximale.

5. Dispositif d'ostéosynthèse selon l'une des revendications 1 à 4, caractérisé en ce que le clou de verrouillage (10) possède, dans sa partie distale, un profil en forme de feuille de trèfle en coupe transversale.

6. Dispositif d'ostéosynthèse selon l'une des revendications 1 à 5, caractérisé en ce que la longueur du clou de verrouillage est comprise entre 150 et 250 mm et est égale de préférence à environ 200 mm.

7. Dispositif d'ostéosynthèse selon l'une des revendications 3 à 6, caractérisé en ce que l'épaisseur de paroi dans la partie creuse du clou de verrouillage (10) correspond au moins à la moitié du diamètre du perçage intérieur.

8. Dispositif de synthèse selon l'une des revendications 2 à 7, caractérisé par le fait que la tige de verrouillage (12) est disposée en renfoncement dans le clou de verrouillage (10).

9. Dispositif d'ostéosynthèse selon l'une des revendications 1 à 8, caractérisé en ce que la vis (11) destinée à être fixée dans le col du fémur possède, dans sa partie terminale, un filetage autotaraudeur (23).

10. Dispositif d'ostéosynthèse selon la revendication 9, caractérisé en ce que deux tranchants en dépouille à arêtes vives (24, 25) diamétralement opposés sont formés dans la partie filetée de la vis destinée à être fixée dans le col du fémur.

## Claims

1. An assisting means for the osteosynthesis for the support of subtrochanteric fractures comprising a femur nail (10) adapted to be introduced into the medulla cavity and a femur neck screw (11) retained and guided in a transverse throughbore (18) of the femur nail, wherein said femur nail (10) is a locking nail (10) adapted to be proximally introduced into the femur, said locking nail having at least one transverse throughbore (14, 15) for the reception of a bone screw, characterized in that said femur neck screw (11), which is adapted to be laterally introduced through said transverse throughbore (18) and has in its end portion a thread coorperating with the femur neck or the femur head, is freely axially displaceable, and in that an anti-rotation means (12) is disposed at said locking nail (10), said anti-rotation means selectively preventing a rotation of said femur neck screw (11) but furthermore allowing an axial movement thereof.

2. The assisting means for osteosynthesis according to claim 1, characterized in that a plurality of grooves (21), which are parallel with respect to the axis and which are circumferencially spaced, are formed in the range of said transverse throughbore (18) at the periphery of said femur neck screw (11), wherein a locking pin (12) engages into said grooves and is adapted to be fixed in the proximal end of said locking nail (10).

3. The assisting means for osteosynthesis according to one of the claims 1 to 2, characterized in that said locking nail (10) is hollow in the proximal portion and is solid or hollow in the distal portion.

4. The assisting means for osteosynthesis according to claim 3, characterized in that the distal portion has a slightly smaller diameter than the proximal portion.

5. The assisting means for osteosynthesis according to one fo the claims 1 to 4, characterized in that said locking nail (10) has a clover leaf profile in cross-section in its distal portion.

6. The assisting means for osteosynthesis according to one of the claims 1 to 5, characterized in that the length of the locking nail is between 150 and 250 mm, preferably about 200 mm.

7. The assisting means for osteosynthesis according to one of the claims 3 to 6, characterized in that the wall thickness in the hollow range of said locking nail (10) corresponds to at least half the diameter of the inner bore.

8. The assisting means for osteosynthesis according to one of the claims 2 to 7, characterized in that said locking pin (12) is disposed in said locking nail (10) in a sunk manner.

9. The assisting means for osteosynthesis according to one of the claims 1 to 8, characterized in that said femur neck screw (11) has a self-cutting thread (23) in its end portion.

10. The assisting means for osteosynthesis according to claim 9, characterized in that two diametrically opposed sharp-edged undercut cutting edges (24, 25) are formed in the thread portion of said femur neck screw.

EP 0 257 118 B1

**FIG.2**

**FIG.3**

**FIG.1**

26

24

25

27

<u>FIG.4</u>

21

<u>FIG.5</u>